(19) Europäisches Patentamt
European Patent Office
Office européen des brevets

(11) Publication number: 0 284 686 A1

# (12) EUROPEAN PATENT APPLICATION

(21) Application number: 87309689.5

(22) Date of filing: 02.11.87

(51) Int. Cl.⁴: C07D 249/18 , A61K 31/41

The title of the invention has been amended (Guidelines for Examination in the EPO, A-III, 7.3).

(30) Priority: 13.11.86 GB 8627170

(43) Date of publication of application: 05.10.88 Bulletin 88/40

(84) Designated Contracting States: BE CH DE FR GB IT LI NL

(71) Applicant: BEECHAM GROUP PLC
Beecham House Great West Road
Brentford Middlesex TW8 9BD(GB)

(72) Inventor: Tedder, John Martin Beecham Pharmaceuticals
Great Burgh Yew Tree Bottom Road
Epsom Surrey, KT18 5XQ(GB)
Inventor: Buckle, Derek Richard Beecham Pharmaceuticals
Great Burgh Yew Tree Bottom Road
Epsom Surrey, KT18 5XQ(GB)

(74) Representative: Russell, Brian John et al
European Patent Attorney Beecham Pharmaceuticals Great Burgh Yew Tree Bottom Road
Epsom Surrey, KT18 5XQ(GB)

## (54) Condensed triazoles, their preparation and their use as medicines.

(57) A compound of formula (I):

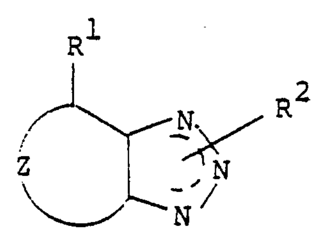

(I)

or a pharmaceutically acceptable salt thereof,
wherein $R^1$ represents hydroxyl or an in-vivo hydrolysable ester thereof; $R^2$ represents a moiety $-(CH_2)_nR^3$ wherein n represents an integer 1 or 2 and $R^3$ represents phenyl or substituted phenyl, and Z represents a residue of a substituted or unsubstituted aryl group; a pharmaceutical composition containing such compounds and the use of such compounds and compositions in medicine.

EP 0 284 686 A1

## CHEMICAL COMPOUNDS

The present invention relates to a class of N-benzyl benzotriazole derivatives, to processes for preparing such compounds, to pharmaceutical compositions containing them and to the use of such compounds and compositions in medicine.

German Patent Number 838,692, (Chemical Abstracts, Volume 51, 11900i) discloses compounds of formula (A):

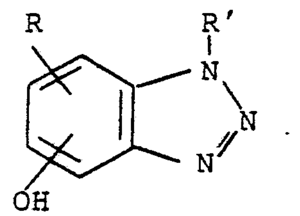

(A)

wherein R is H, alkyl, alkoxy, HOOC or OH and R′ indicates H, $PhCH_2$ or alkyl containing at most 3 C atoms. Such compounds are disclosed as being useful as azo components in the preparation of light-sensitive layers for diazotypes.

It has now surprisingly been discovered that a class of N-benzyl benzotriazole derivatives are active as inhibitors of the enzyme 5-lipoxygenase, an important enzyme in the production of the spasmogenic peptide leukotrienes($LTC_4$, $LTD_4$, $LTE_4$, collectively the slow reacting substance of anaphylaxis, SRS-A) and inflammatory leukotriene $LTB_4$. These compounds are also active at inhibiting SRS-A production in vivo and are thus of value in the prophylaxis and treatment of diseases whose symptoms are controlled by these mediators such as asthma, rheumatoid arthritis, psoriasis and other allergic and inflammatory disorders.

Accordingly, the present invention provides a compound of formula (I):

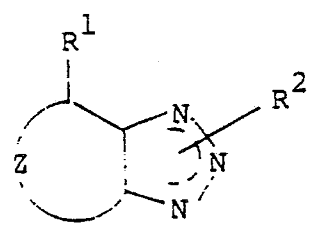

(I)

or a pharmaceutically acceptable salt thereof, wherein $R^1$ represents hydroxyl or an in-vivo hydrolysable ester thereof; $R^2$ represents a moiety $-(CH_2)_nR^3$ wherein n represents an integer 1 or 2 and $R^3$ represents phenyl or substituted phenyl, and Z represents a residue of a substituted or unsubstituted aryl group.

A suitable residue of a substituted or unsubstituted aryl group includes a residue of a substituted or unsubstituted phenyl or naphthyl group.

Favourably, Z represents a residue of a substituted or unsubstituted phenyl group.

Preferably, Z represents a residue of a substituted phenyl group.

Suitable substituents for any aryl group include up to five, preferably up to three, groups selected from halogen; alkyl; phenyl; alkoxy; acyloxy; haloalkyl; hydroxy or an in-vivo hydrolysable ester thereof; or a pharmaceutically acceptable salt thereof; amino; nitro; carboxy or a pharmaceutically acceptable salt, amide or ester thereof; or a thioalkyl group.

A preferred aryl substituent is an alkyl group

Suitably, n represents an integer 1.

In one preferred aspect the present invention provides a compound of formula (II):

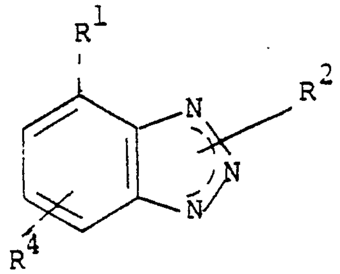

(II)

or a pharmaceutically acceptable salt thereof, wherein $R^1$ and $R^2$ are as defined in relation to formula (I) and $R^4$ represents a hydrogen or halogen atom or an alkyl, alkoxy, acyloxy, thioalkyl or nitro group.

Preferably, $R^1$ represents a hydroxyl group.

Suitably, $R^2$ is attached to the N-1 or N-2 nitrogen atom of the triazole ring, favourably $R^2$ is attached to the N-2 nitrogen atom of the triazole ring.

Favourably, $R^2$ is attached to the N-3 nitrogen atom of the triazole ring.

Suitably, $R^2$ represents a moiety $-CH_2R^3$.

Favourably, $R^3$ represents phenyl or methoxyphenyl, thus favourably $R^2$ represents a benzyl group or a methoxybenzyl group.

Preferably, $R^2$ represents a benzyl group.

Suitably, $R^4$ is located on the phenyl ring at the para position relative to $R^1$.

Suitably, $R^4$ represents an alkyl group.

Preferably, $R^4$ represents a methyl group.

In a particularly preferred aspect the present invention provides 3-benzyl-4-hydroxy-7-methylbenzotriazole or an in-vivo hydrolysable ester thereof, and/or or a pharmaceutically acceptable salt thereof.

In a particularly preferred aspect the present invention provides 2-benzyl-4-hydroxy-7-methylbenzotriazole, or an in-vivo hydrolysable ester thereof and/or a pharmaceutically acceptable salt thereof.

Suitable pharmaceutically acceptable salts include salts of hydroxyl groups and any carboxyl group present in the compound of formula (I).

Suitable pharmaceutically acceptable salts of the carboxyl groups of the compounds of formula (I) include metal salts, such as for example aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabietylamine, N,N′-bisdehydroabietylamine, glucamine, N-methylglucamine or bases of the pyridine type such as pyridine, collidine or quinoline. Suitable pharmaceutically acceptable salts of hydroxyl groups include metal salts especially alkali metal salts such as sodium or potassium salts.

Suitable pharmaceutically acceptable amides of the carboxyl groups of the compounds of formula (I) include amides of formula $-CONR^sR^t$ wherein $R^s$ and $R^t$ each independently represents hydrogen or $C_{1-6}$ alkyl; or $R^s$ and $R^t$ together with the nitrogen to which they are attached form a saturated 5-or 6-membered ring.

Suitable pharmaceutically acceptable esters of the carboxyl groups of the compounds of formula (I), include in-vivo hydrolysable esters.

When used herein the term "in-vivo hydrolysable ester" relates to a pharmaceutically acceptable ester which readily breaks down in the human or non-human animal body to leave the free carboxyl group or hydroxy group, or a salt thereof.

Suitable in vivo hydrolysable carboxyl esters include those provided by $C_{1-6}$ alkyl groups, $C_{1-6}$ alkyl groups substituted by a hydroxyl group not on the α-carbon atom and groups of the sub-formulae (a) or (b)

$-CHR^5-O-CO-R^6$ (a)

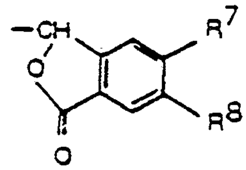

(b)

wherein $R^5$ is a hydrogen atom or a methyl group; $R^6$ is a $C_{1-6}$ alkyl or phenyl group; $R^7$ is a hydrogen atom or a methyl or methoxyl group; and $R^8$ is a hydrogen atom or a methyl or methoxyl group.

Particularly suitable esters include the methyl, ethyl, propyl and butyl esters, for example the methyl ester the ethyl ester and the isopropyl ester.

Suitable in-vivo hydrolysable hydroxyl esters are those provided by $C_{1-6}$ alkyl carboxylic acids.

The present invention encompasses all suitable combinations of pharmaceutically acceptable salts, esters and amides obtainable by conventional means from the compounds of formula (I).

When used herein the term "alkyl" or the term "alk" (as used for example in "alkoxy"), relates to a straight or branched chain alkyl group of up to 12 carbon atoms, suitably from 1 to 6 carbon atoms, for example methyl, ethyl, propyl or butyl groups.

When used herein the term "halogen" or "halo" refers to fluorine, chlorine bromine or iodine.

The term "N-2 nitrogen atom of the triazole ring" and the like terms relate to the following numbering for the nitrogen atoms of the triazole ring in the compounds of formula (I):

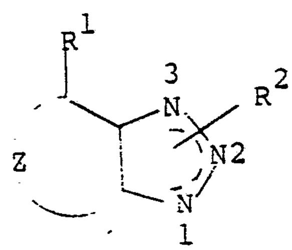

The present invention also provides a process for preparing a compound of the hereinbefore defined formula (I), or a pharmaceutically acceptable salt thereof, which process comprises reacting a compound of formula (III):

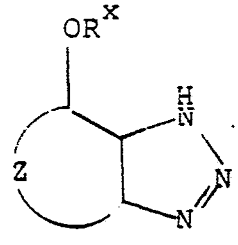

(III)

wherein Z is as defined in relation to formula (I) and $R^x$ represents hydrogen or an hydroxyl protecting group, with a compound of formula (IV):

$R^2$-X (IV)

wherein $R^2$ is as defined in relation to formula (I) and X represents a leaving group;
and thereafter, if necessary, separating the required compound of formula (I) from any other compound of formula (I) and/or removing any protecting group $R^x$ and/or, if required, preparing an in-vivo hydrolysable ester of the compound of formula (I) and/or a pharmaceutically acceptable salt thereof.

Suitable hydroxyl protecting groups $R^x$ are those used conventionally in the art such as for example a methyl, benzyl or tosyl group.

Suitably, X represents a halogen such as chloro, bromo or iodo.

The reaction between the compounds of formula (III) and (IV) may be carried out in any suitable solvent, for example N,N-dimethylformamide, at low to medium temperatures, such as a temperature in the range of 10-100°C.

The abovementioned separation of a compound of formula (I) from any other compound of formula (I) includes the separation of those isomeric compounds of formula (I) wherein the moiety $R^2$ is attached to the N-1, N-2 or N-3 nitrogen atoms.

The compounds of formula (I) prepared in the above reaction including the said isomeric compounds of formula (I) may be separated from each other using conventional separation techniques such as chromatography, particularly column chromatography.

The compounds of formula (III) may be prepared from compounds of formula (V):

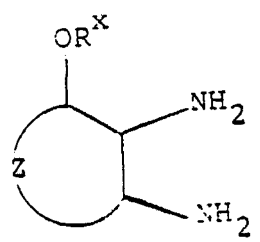

(V)

wherein Z and $R^x$ are as defined in relation to formula (III), by reaction with a source of nitrous acid.

A suitable source of nitrous acid is alkali metal nitrites (eg. sodium or potassium nitrite) in dilute aqueous acids such as dilute hydrochloric or dilute sulphuric acids.

The preceding reaction may be carried out in any suitable solvent, for example ethanolic dilute hydrochloric acid at a temperature range of between -20 to 20°C, conveniently at 0-5°C.

The compounds of formulae (IV) and (V) are either known compounds or may be prepared by methods analogous to those used to prepare known compounds or they are available commercially; for example using the methods or analogous methods to those disclosed in European Published Application Number 0,178,413: Thus a compound of formula (V) may be prepared by catalytic reduction of the corresponding dinitro compound.

The present invention also provides a compound of formula (I), or a pharmaceutically acceptable salt thereof, for use as an active therapeutic substance, particularly for use in the treatment and/or prophylaxis of allergic diseases, such as asthma, hayfever, rhinitis or allergic eczema, or of inflammatory conditions such as arthritis and psoriasis.

The compounds of formula (I) or a pharmaceutically acceptable salt thereof, may be used alone or in the form of a pharmaceutical composition. Suitably they are used in the form of a pharmaceutical composition.

Accordingly, the present invention also provides a pharmaceutical composition comprising a non-toxic amount of a compound of formula (I) or a pharmaceutically acceptable salt thereof, and a pharmaceutically acceptable carrier therefor.

Preferably, pharmaceutical compositions are in unit dosage form and in a form adapted for use in the medical or veterinarial fields. For example, such compositions may be in a pack form accompanied by written or printed instructions for use as an agent in the treatment or prophylaxis of any of the disorders mentioned above.

The suitable dosage range for the compounds of the invention may vary from compound to compound and may depend on the condition to be treated. It will also depend, inter alia, upon the relation of potency to absorbability and the mode of administration chosen. The compound or composition of the invention may be formulated for administration by any route, the preferred route depending upon the disorder for which treatment is required, and is preferably in unit dosage form or in a form that a human patient may administer to himself in a single dosage. Advantageously, the composition is suitable for oral, rectal, topical, parenteral, intravenous or intramuscular administration or through the respiratory tract. Preparations may be designed to give slow release of the active ingredient.

Compositions may, for example, be in the form of tablets, capsules, sachets, vials, powders, granules, lozenges, reconstitutable powders, or liquid preparations, for example solutions or suspensions, or suppositories. Compositions which are especially suitable for administration to the respiratory tract and for topical administration are discussed in more detail below.

The compositions, for example those suitable for oral administration, may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable setting agents such as sodium lauryl sulphate.

Solid compositions may be obtained by conventional methods of blending, filling tabletting or the like. Repeated blending operations may be used to distribute the active agent throughout those compositions employing large quantities of fillers. When the composition is in the form of a tablet, powder, or lozenge, any carrier suitable for formulating solid pharmaceutical compositions may be used, examples being magnesium stearate, starch, glucose, lactose, sucrose, rice flour and chalk. Tablets may be coated according to methods well known in normal pharmaceutical practice, in particular with an enteric coating. The composition may also be in the form of an ingestible capsule, for example of gelatin containing the compound, if desired with a carrier or other excipients.

Compositions for oral administration as liquids may be in the form of, for example, emulsions, syrups, or elixirs, or may be presented as a dry product for reconstitution with water or other suitable vehicle before use. Such liquid compositions may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; aqueous or non-aqueous vehicles, which include edible oils, for example almond oil, fractionated coconut oil, oily esters, for example esters of glycerine, or propylene glycol, or ethyl alcohol, glycerine, water or normal saline; preservatives, for example methyl or propyl p-hydroxybenzoate or sorbic acid; and if desired conventional flavouring or colouring agents.

The compounds of this invention may also be administered by a non-oral route. In accordance with routine pharmaceutical procedure, the compositions may be formulated, for example for rectal administration as a suppository or for presentation in an injectable form in an aqueous or non-aqueous solution, suspension or emulsion in a pharmaceutically acceptable liquid, e.g. sterile pyrogen-free water or a parenterally acceptable oil or a mixture of liquids, which may contain bacteriostatic agents, anti-oxidants or other preservatives, buffers or solutes to render the solution isotonic with the blood, thickening agents, suspending agents or other pharmaceutically acceptable additives. Such forms will be presented in unit dose form such as ampoules or disposable injection devices or in multi-dose forms such as a bottle from which the appropriate dose may be withdrawn or a solid form or concentrate which can be used to prepare an injectable formulation.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns. Where appropriate, small amounts of other anti-asthmatics and bronchodilators, for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included. For topical administration, compositions may be presented as an ointment, cream, lotion, gel, gel stick, spray, aerosol, or skin paint.

By way of example, in any of the preceding formulations a suitable dosage unit might contain 0.01 to 500mgs of active ingredient, more suitably 1 to 500mgs via the oral route, 0.01 to 10mgs via inhalation. The effective dose of compound depends on the particular compound employed, the condition of the patient and on the frequency and route of administration, but in general is in the range of from 0.001 mg/kg/day to 100 mg/kg/day inclusive of the patient's body weight. For use in treatment of inflammatory disorders a composition of the invention will preferably be in a form suitable for oral administration, for example a tablet or capsule or a sachet containing reconstitutable powder. A unit dose will generally contain from 20 to 1000 mg and preferably will contain from 30 to 500 mg, in particular 50, 100, 150, 200, 250, 300, 350, 400, 450, or 500 mg. The composition may be administered once or more times a day for example 2, 3 or 4 times daily, and the total daily dose for a 70 kg adult will normally be in the range 100 to 3000 mg. Alternatively the unit dose will contain from 2 to 20 mg of active ingredient and be administered in multiples, if desired, to give the preceding daily dose.

In a further aspect the present invention also encompasses a method for the treatment and/or prophylaxis of allergic diseases, such as asthma, hay fever, rhinitis or allergic eczema, or of inflammatory

conditions such as arthritis and psoriasis, which method comprises the administration of an effective, non-toxic amount of a compound of formula (I) or a pharmaceutically acceptable salt thereof, to a human or non-human mammal in need thereof.

The invention also comprises the use of a compound of formula (I) or a pharmaceutically acceptable salt thereof, for the manufacture of a medicament for the treatment and/or prophylaxis of allergic diseases, such as asthma, hayfever, rhinitis or allergic eczema, or of inflammatory conditions such as arthritis and psoriasis.

Within the above mentioned dosage ranges, no adverse toxicological effects are indicated with the compounds of the invention.

Compounds of this invention, their preparation and their biological activity are illustrated in the following Examples and Pharmacological Data.

Example 1

Preparation of 3-Benzyl-4-hydroxy-7-methylbenzotriazole

3-Benzyl-4-benzyloxy-7-methylbenzotriazole (100mg 0.30mmole) was dissolved in ethanol (50ml) and hydrogenated over palladium on charcoal (10%, 20mg) until it had absorbed the theoretical amount of hydrogen. The mixture was filtered and the filtrate evaporated to yield the title compound (55mg 75.7%) m.p. 212°C,

NMR (DMSO) δ:2.60(s,3H); 6.10(s,2H); 6.73(d,IH, J=7.5Hz);
7.0(d,IH,J=7.5Hz); 7.30(s,5H) 10.37(s,IH).

Infra-red:ν 2720, 2600, 1600, 1535, 1070, 820, 725, 700

Elemental Analysis (%):Found: C:70.16, H:5.54, N:17.42;
Calculated: C:70.27, H:5.48, N:17.56.

Example 2

Preparation of 2-Benzyl-4-hydroxy-7-methylbenzotriazole

This was prepared from the corresponding 0-benzyl compound by the method given in Example 1. Yield 73.2% m.p. 149°C,

NMR (DMSO) δ: 2.36(s,3H); 6.0(s,2H); 6.67(d,1H, J=7.5H); 7.00(d,1H,J=7.5Hz) 7.37; (s,5H); 10.23(s,1H).

Infra-red:ν 3350, 3260, 1600, 1530, 820, 720, 700.

Elemental Analysis:Found: C: 70.22, H:5.54, N:17.52;
Calculated: C:70.27, H:5.48, N:17.56.

Example X1

Preparation of N-Benzyl-4-benzyloxy-7-methylbenzotriazole isomers

4-Benzyloxy-7-methylbenzotriazole (239mg, 1mmole) was combined with benzyl bromide (171mg, 1mmole) excess potassium carbonate (300mg), and N,N-dimethylformamide (20ml). The mixture was stirred for 24 hours, then poured into water (200ml) and extracted with ethyl acetate (3×40ml). The combined extracts were washed with water (2×50ml) dried and evaporated to yield a white solid (329mg). This was a mixture of two isomers which was separated by chromatography on silica eluting with chloroform. 2-Benzyl-4-benzyloxy-7-methylbenzotriazole eluted first (178mg, 54%) followed by 3-benzyl-4-benzyloxy-7-methylbenzotriazole (132mg 42%).

The 2-benzyl isomer had the following physical properties:

m.p. 88-89°C,

NMR (DMSO) δ:2.47(s,3H); 5.30(s,2H); 5.9(s,2H); 6.73(1H,d, J=7.5Hz);7.01(1H,d, J=7.5Hz); 7.4(10H,m).

Infra-red:ν 1600, 1530, 810, 725, 690

Elemental Analysis (%)Found: C:76.40, H:5.61, N:12.73;
Calculated: C:76.57, H:5.84. N:12.76.

The 3-benzyl isomer had the following physical properties:
m.p. 101-2°C,

NMR(DMSO) δ: 2.56(s,3H); 5.27(s,2H); 5.97(s,2H); 7.2(bm,7H); 7.4(s,5H).

Infra-red:ν 1600, 1530, 1080, 790, 760, 680.

Elemental Analysis (%)Found C:76.71, H:5.55, N:12.60;
Calculated: C:76.57, H:5.84, N:12.76.

Example X2

Preparation of 4-benzyloxy-7-methylbenzotriazole

1-Benzyloxy-2,3-dinitro-4-methylbenzene (4.0g 13.9mmole) was hydrogenated in ethanol (100ml) over Raney Nickle (1.6g) until it had absorbed six moles of hydrogen. The mixture was filtered into hydrochloric acid (200ml) and cooled to 0-5°C. A solution of sodium nitrite (8.5g in 25ml water) was added and the mixture allowed to stand overnight. The product was filtered off and recrystallized from methanol/chloroform to give buff coloured crystals of the title compound m.pt 234-5°C (840mg 26.2%).

NMR (DMSO) δ:2.55(s,3H); 5.37(s,2H); 6.85(1H,d, J=7.5Hz);
7.10(1H,d, J=7.5Hz); 7.50 (5H,m).

Infra-red:ν 3100, 1610, 1535, 800, 770, 740, 700

Elemental Analysis (%)Found: C:70.20, H:5.38, N:17.48
Calculated: C:70.27, H:5:48, N:17.56

## BIOLOGICAL DATA

### 1) RBL-1 5-LIPOXYGENASE SCREEN

5-Lipoxygenase enzyme was prepared as a 10,000g supernatant from RBL-1 cells by the method of Jakschik (Jakschik, B.A., F.F. Sun, L.M.Lee, and M.M. Steinhoff, 1980, Biochem. Biophys. Res.Comm. 95 103). The 10,000g supernatant was diluted with homogenization buffer to the equivalent of 1.5 - 2.5 $\times$ $10^7$ cells. $ml.^{-1}$ and made 2mM with respect to $CaCl_2$. Aliquots of 0.5ml were then dispensed into tubes, and incubated at 29°C with 5µl ethanol or compound in ethanol at the desired concentration for 2min. Then [1-$^{14}$C] arachidonic acid was added in buffer to give a final concentration of 6.3µM and 0.2µCi per incubation, and the reaction continued at 29°C for 2min. The reaction was terminated by adding 1ml of acetone and cooling on ice, 0.5ml of ice-cold saline and 100µl of 2N formic acid were added, and the mixture was extracted with 2 $\times$ 2ml of chloroform. The extract was stored under $N_2$ at -20°C until analysis by chromatography. Activity was measured as the percentage of total radioactivity found in 5-HETE and 5,12-diHETE, and inhibition calculated as the decrease in formation of the sum of these two species in compound-treated incubates relative to control incubations.

### 2) RAT PASSIVE PERITONEAL ANAPHYLAXIS (PPA)

The method is essentially similar to that described previously by Ross et al (Ross, Janet W., Smith, H. and Spicer, Barbara A. Increased vascular permeability during passive peritoneal anaphylaxis in the rat. Int. Arch. Allergy appl. Immun. 51, 226, 1976.)

#### Animals

Charles River Sprague Dawley male rats of 225-275 g and Dunkin Hartley male white guinea pigs of 250-300 g were used.

#### Antiserum

Charles River Sprague Dawley male rats of 225 - 275 g were given intraperitoneal injections of 0.5 ml of either Bordetella pertussis vaccine (4 $\times$ $10^{10}$ organisms/ml; Burroughs Wellcome, London) or Pertussis vaccine absorbed (not less than 41.u. B. pertussis antigen with aluminium hydroxide; The Lister Institute Elstree, England), and subcutaneous injections of 0.5ml of an emulsion of 100 mg of ovalbumin (chicken egg albumin; crystallised and lyophilised, grade V, Sigma, London) in 2ml of isotonic saline and 3ml of incomplete Freund's adjuvant. The rats were bled by cardiac puncture on day 18, the blood was pooled and the serum separated, stored at -20°C and thawed only once before use. The serum produced 72 hour passive cutaneous anaphylaxis activity in recipient rats to a dilution of 1:64 to 1:32 which was decreased to a dilution of less than 1:2 by heating at 56°C for 4 hours.

#### Passive peritoneal anaphylaxis

Rats were given intraperitoneal injections of 2 ml of a 1:5 dilution of the rat anti-serum in isotonic saline. Two hours later 0.3ml of 5% solution of Pontamine Sky Blue (Raymond A. Lamb, London) in isotonic saline was injected intraveneously, followed by an intraperitoneal injection of the test compound in 1 ml of saline; (control rats received 1 ml of saline); followed 2.5 minutes later by an intraperitoneal injection of 5 ml of a Tyrode solution containing 50µg/ml heparin and 0.4 mg/ml of ovalbumin. The concentrations of the compounds were quoted as that in the 6 ml of fluid injected intraperitoneally. Exactly 5 minutes after challenge the rats were stunned and bled and their peritoneal fluids were collected by opening their

peritoneal cavities over funnels into polycarbonate tubes in ice. The supernatants were separated from the cellular residue by centrifuging at 150 g for 5 minutes and any samples obviously contaminated with blood were discarded for estimation of dye, histamine and SRS-A. Groups of at least 5 rats were used for each dose of compound and the treatments were randomized.

Assay of peritoneal fluids

Collected peritoneal fluids were centrifuged and the supernatant fluids assayed for dye immediately. 0.5 ml samples of the supernatants were added to 1 ml of 12% trichloracetic acid and stored at -20°C and used to assay for histamine. The remainders of the supernatant fluids were placed in a boiling water bath for 5 minutes and stored frozen at -20°C until assayed for SRS-A.

Dye Assay

The optical densities (OD) of the supernatants which were not contaminated with blood were determined immediately at 625nm.

Histamine Assay

Histamine was assayed using an automated spectrofluorimetric system (technicon Autoanalyser) by a method similar to that of Evans, D.P., Lewis, J.A. and Thomson, D.S.: (An automated fluorimetric assay for the rapid determination of histamine in biological fluids. Life Sci. 12, 327, 1973). At the concentrations used the compounds tested did not interfere with the assay.

SRS-A Assay

SRS-A was assayed on the isolated guinea pig ileum preparation in the presence of atropine ($5 \times 10^{-7}$ M) and mepyramine maleate ($10^{-6}$ M), the latter to abolish the histamine response. (Brocklehurst, W.E. The release of histamine and formation of a slow reacting substance (SRS-A) during anaphylactic shock. J. Physiol., Lond. 151, 416, 1960). Bulked peritoneal fluids from passively sensitised and challenged rats were centrifuged, heated, stored at -20°C in 0.5 ml aliquots, and used as a reference SRS-A standard, and arbitrarily designated as containing 10 Units per ml. Concentrations of the unknown were bracketed by reference SRS-A samples. At the concentrations used, the compounds tested did not interfere with the assay.

BIOLOGICAL RESULTS

1. RBL-1 5-Lipoxygenase Screen

| Example No. | Percentage inhibition of RBL-1 5-lipoxygenase at 20μM |
|---|---|
| 1 | 89 |
| 2 | 81 |

2. Rat passive peritoneal anaphylaxis

All compounds were injected i.p. to give a final concentration of $2 \times 10^{-4}$ M with a contact time of 2½ minutes.

| Example No. | Percentage inhibition Dye | Histamine | SRS-A |
|---|---|---|---|
| 2 | 30 | 45 | 49 |

## Claims

1. A compound of formula (I):

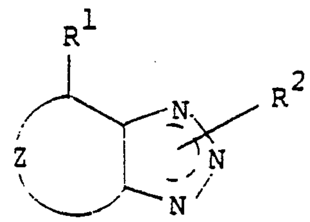

(I)

or a pharmaceutically acceptable salt thereof, characterized in that, $R^1$ represents hydroxyl or an in-vivo hydrolysable ester thereof; $R^2$ represents a moiety $-(CH_2)_nR^3$ wherein n represents an integer 1 or 2 and $R^3$ represents phenyl or substituted phenyl, and Z represents a residue of a substituted or unsubstituted aryl group.

2. A compound according to claim 1, wherein Z represents a residue of a substituted or unsubstituted phenyl or naphthyl group.

3. A compound according to claim 1 or claim 2, wherein Z represents a substituted phenyl group.

4. A compound according to any one of claims 1 to 3, of formula (II):

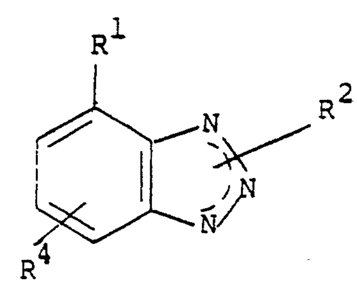

(II)

or a pharmaceutically acceptable salt thereof, wherein $R^1$ and $R^2$ are as defined in relation to formula (I) and $R^4$ represents a hydrogen or halogen atom or an alkyl, alkoxy, acyloxy, thioalkyl or nitro group.

5. A compound according to any one of claims 1 to 4, wherein $R^1$ represents a hydroxyl group.

6. A compound according to any one of claims 1 to 5, wherein $R^2$ represents a benzyl group.

7. A compound according to any one of claims 4 to 6, wherein $R^4$ represents an alkyl group.

8. A compound according to claim 1, selected from:
3-benzyl-4-hydroxy-7-methyl-benzotriazole; and

2-benzyl-4-hydroxy-7-methyl-benzotriazole; or an in-vivo hydrolysable ester thereof and/or a pharmaceutically acceptable salt thereof.

9. A process for preparing a compound of formula (I), according to claim 1, or a pharmaceutically acceptable salt thereof, which process comprises reacting a compound of formula (III):

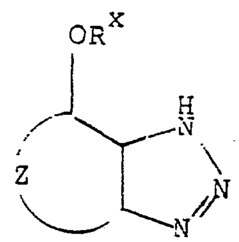

(III)

wherein Z is as defined in relation to formula (I) and $R^x$ represents hydrogen or a hydroxyl protecting group, with a compound of formula (IV):

$R^2$-X (IV)

wherein $R^2$ is as defined in relation to formula (I) and X represents a leaving group;
and thereafter, if necessary, separating the required compound of formula (I) from any other compound of formula (I) and/or removing any protecting group $R^x$ and/or, if required, preparing an in-vivo hydrolysable ester of the compound of formula (I) and/or a pharmaceutically acceptable salt thereof.

10. A compound of formula (I), as defined in claim 1, or a pharmaceutically acceptable salt thereof, for use as an active therapeutic substance.

11. A compound of formula (I), as defined in claim 1, or a pharmaceutically acceptable salt thereof, for use in the treatment and/or prophylaxis of allergic diseases and/or of inflammatory conditions.

12. A pharmaceutical composition comprising a non-toxic amount of a compound of formula (I), as defined in claim 1, or a pharmaceutically acceptable salt thereof, and a pharmaceutically acceptable carrier therefor.

13. The use of a compound of formula (I), as defined in claim 1, or a pharmaceutically acceptable salt thereof, for the manufacture of a medicament for the treatment and/or prophylaxis of allergic diseases and/or of inflammatory conditions.

European Patent Office

# EUROPEAN SEARCH REPORT

Application Number

EP 87 30 9689

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 002 310 (BEECHAM)<br>* Whole document *<br>--- | 1-13 | C 07 D 249/18<br>A 61 K 31/41 |
| Y | EP-A-0 178 413 (BEECHAM)<br>* Whole document *<br>--- | 1-13 | |
| D,A | DE-C- 838 692 (KALLE)<br>* Whole document *<br>----- | 1-13 | |
| | | | **TECHNICAL FIELDS SEARCHED (Int. Cl.4)** |
| | | | C 07 D 249/00 |

The present search report has been drawn up for all claims

| Place of search | Date of completion of the search | Examiner |
|---|---|---|
| THE HAGUE | 19-02-1988 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)